(19) Europäisches Patentamt
European Patent Office
Office européen des brevets

(11) **EP 4 501 319 A1**

(12) **EUROPEAN PATENT APPLICATION**
published in accordance with Art. 153(4) EPC

(43) Date of publication:
**05.02.2025  Bulletin 2025/06**

(21) Application number: 23780513.0

(22) Date of filing: **28.03.2023**

(51) International Patent Classification (IPC):
*A61K 9/44* (2006.01)       *A61K 9/30* (2006.01)
*A61K 47/02* (2006.01)      *A61K 47/10* (2017.01)
*A61K 47/12* (2006.01)      *A61K 47/20* (2006.01)
*A61K 47/22* (2006.01)      *A61K 47/24* (2006.01)
*A61K 47/26* (2006.01)      *A61K 47/36* (2006.01)
*A61K 47/38* (2006.01)      *B41J 2/01* (2006.01)
*B41M 5/00* (2006.01)       *C09D 11/30* (2014.01)

(52) Cooperative Patent Classification (CPC):
**A61K 9/2072; A61K 9/28; A61K 47/02;
A61K 47/10; A61K 47/12; A61K 47/20;
A61K 47/22; A61K 47/24; A61K 47/26;
A61K 47/36; A61K 47/38; B41J 2/01; B41M 5/00;
C09D 11/30**

(86) International application number:
**PCT/JP2023/012488**

(87) International publication number:
**WO 2023/190504 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **31.03.2022  JP 2022059782**

(71) Applicant: **Toppan Holdings Inc.**
**Tokyo 110-0016 (JP)**

(72) Inventors:
• **KOSEKI Seiya**
**Tokyo 110-0016 (JP)**
• **HASHIMOTO Risa**
**Tokyo 110-0016 (JP)**
• **MIYAZAWA Yasuhisa**
**Tokyo 110-0016 (JP)**
• **HOSHINO Yuichi**
**Tokyo 110-0016 (JP)**
• **SAITO Masatoshi**
**Tokyo 110-0016 (JP)**
• **UCHIYAMA Hiromitsu**
**Tokyo 110-0016 (JP)**

(74) Representative: **TBK**
**Bavariaring 4-6**
**80336 München (DE)**

(54) **TABLET, PRINTED MATTER, AND PRINTING METHOD FOR EDIBLE INK**

(57)     An object of the present invention is to provide an edible ink printing method that can sufficiently prevent the light discoloration and light fading of printed images and improve the light fastness of the printed images, a tablet comprising a printed part printed using the printing method, and a printed matter comprising a printed part printed using the printing method. The edible ink printing method according to the present embodiment comprises performing thinning printing so that when a ratio of the total area of a plurality of dots (b) printed while performing thinning printing to the area of a solid printed part (A) printed without performing thinning printing (total area of dots (b) printed while performing thinning printing/area of solid printed part (A) printed without performing thinning printing) is defined as a printing area ratio, the printing area ratio is within the range of 30% or more and 80% or less, an ink used for printing being an edible IJ ink, and the edible IJ ink comprising an edible dye.

EP 4 501 319 A1

# FIG.1

(a)

A

1

(b)

b
b } B

C

1

(c)

AREA RATIO 100%

A

INK

SUBSTRATE (TABLET) — 1

⬇ LIGHT IRRADIATION

80% DECREASE

SUBSTRATE (TABLET) — 1

DECREASE RATE = 100% × 80%
= 80%

(d)

AREA RATIO 60%

B

b

SUBSTRATE (TABLET) — 1

⬇ LIGHT IRRADIATION

80% DECREASE

b

SUBSTRATE (TABLET) — 1

DECREASE RATE = 60% × 80%
= 48%

**Description**

[Technical Field]

The present invention relates to a tablet, a printed matter, and an edible ink printing method.

[Background Art]

Examples of inkjet printing inks (hereinafter also referred to simply as "IJ inks") include inkjet inks that are edible by using food dyes, such as tar colorants, as coloring materials (hereinafter also referred to simply as "edible IJ inks").
Conventionally, some edible IJ inks may undergo discoloration due to the deterioration of coloring materials, causing changes in the color (color tone) of the coloring materials in printed characters or images (hereinafter collectively referred to as "printed images"), or fading due to a decrease in the chroma-level of the coloring materials.
For example, when printing images onto tablets or other materials to be printed, color-developing dyes have been used as coloring materials for edible IJ inks (dye inks) for the purpose of improving visibility. However, dyes deteriorate due to decomposition caused by the action of light (photolysis), which may cause discoloration and fading due to light, in printed images.
In recent years, technology development is underway to prevent the discoloration and fading of printed images, and various methods for preventing discoloration and fading have been proposed (e.g., PTL 1). However, conventional methods have not sufficiently prevented light discoloration and light fading of printed images, and have not improved the light fastness of edible IJ inks. More specifically, when a printed image was formed by so-called "solid printing," which is generally used in the prior art, even if the above-mentioned various methods for preventing discoloration and fading were applied, the value of "ΔE," which is an index indicating the degree of light fastness (discoloration and fading), was large, light discoloration and light fading of the printed image could not be sufficiently prevented, and light fastness of the printed image could not be improved in some cases.
Thus, the prior art aimed at preventing discoloration and fading of printed images was based on the premise of performing solid printing. That is, the technical idea (design idea) of the prior art was to prevent discoloration and fading of the entire printed image by adjusting the composition of the edible IJ ink etc., on the premise of performing solid printing.

[Citation List]

[Patent Literature]

PTL 1: JP 6389506 B

[Summary of the Invention]

[Technical Problem]

The present invention has been made in view of these points, and an object of the present invention is to provide an edible ink printing method that can sufficiently prevent light discoloration and light fading of printed images and improve the light fastness of the printed images, a tablet comprising a printed part printed using the printing method, and a printed matter comprising a printed part printed using the printing method.
More specifically, the present inventors have found that "thinning printing," which has been used in the prior art, itself is very effective for the improvement of light fastness, and thus have arrived at the present invention. The present invention is significantly different in terms of perspective from the technical idea (design idea) of the prior art, that is, the technical idea (design idea) of preventing discoloration and fading of the entire printed image by adjusting the composition of the edible IJ ink etc. on the premise of performing solid printing, and discloses a technical idea (design idea) of being capable of preventing the discoloration and fading of the entire printed image by adjusting the printing conditions of thinning printing without adjusting the composition of the edible IJ ink etc.

[Solution to Problem]

In order to achieve the above object, the tablet according to an embodiment of the present invention is characterized in that when a ratio of the total area of a plurality of printed parts printed while performing thinning printing to the area of a printed part printed without performing thinning printing (total area of printed parts printed while performing thinning printing/area of printed part printed without performing thinning printing) is defined as a printing area ratio, the printing area ratio is within the range of 30% or more and 80% or less, an ink used for printing is an edible ink, and the edible ink comprises an edible dye.

Further, in order to achieve the above object, the printed matter according to an embodiment of the present invention is characterized in that when a ratio of the total area of a plurality of printed parts printed while performing thinning printing to the area of a printed part printed without performing thinning printing (total area of printed parts printed while performing thinning printing/area of printed part printed without performing thinning printing) is defined as a printing area ratio, the printing area ratio is within the range of 30% or more and 80% or less, an ink used for printing is an edible ink, and the edible ink comprises an edible dye.

Further, in order to achieve the above object, the edible ink printing method according to an embodiment of the present invention is characterized by comprising performing thinning printing so that when a ratio of the total area of a plurality of printed parts printed while performing thinning printing to the area of a printed part printed without performing thinning printing (total area of printed parts printed while performing thinning printing/area of printed part printed without performing thinning printing) is defined as a printing area ratio, the printing area ratio is within the range of 30% or more and 80% or less, an ink used for printing being an edible ink, and the edible ink comprising an edible dye.

[Advantageous Effects of the Invention]

According to an embodiment of the present invention, the light discoloration and light fading of printed images can be sufficiently prevented, and the light fastness of the printed images can be improved.

[Brief Description of the Drawings]

Fig. 1 is a schematic diagram for explaining an example of an edible IJ ink printing method according to an embodiment of the present invention.
Fig. 2 is a schematic cross-sectional view illustrating an example of a tablet (uncoated tablet) according to an embodiment of the present invention.
Fig. 3 is a schematic cross-sectional view illustrating an example of a tablet (film-coated tablet) according to an embodiment of the present invention.
Fig. 4 is an example of a printed image provided on a tablet (uncoated tablet) according to an embodiment of the present invention.
Fig. 5 is an example of a printed image provided on a tablet (film-coated tablet) according to an embodiment of the present invention.

[Description of the Embodiments]

The edible IJ ink (edible ink) according to an embodiment of the present invention relates to an edible IJ ink that can sufficiently prevent light discoloration and light fading and can improve light fastness of images or the like printed on the surface of pharmaceutical tablets by an inkjet printing method. The configurations of an edible IJ ink printing method according to an embodiment of the present invention, an edible IJ ink used in the printing method, a tablet comprising a printed part printed with the edible IJ ink, and a printed matter comprising a printed part printed with the edible IJ ink will be described in detail below.

[Printing method]

The present inventors found that as an edible IJ ink printing method, when a ratio of the total area of a plurality of printed parts printed while performing thinning printing to the area of a printed part printed without performing thinning printing (total area of printed parts printed while performing thinning printing/area of printed part printed without performing thinning printing) is defined as a printing area ratio, thinning printing is performed so that the printing area ratio is within the range of 30% or more and 80% or less, whereby the light discoloration and light fading of the printed image can be sufficiently prevented. As a result, the present inventors have realized that thinning printing is performed with an edible IJ ink so that the printing area ratio is within the range of 30% or more and 80% or less, whereby the light discoloration and light fading of the printed image can be sufficiently prevented, and the light fastness of the printed image can be improved. That is, in the prior art, improvement in the light fastness of printed images was achieved by appropriately adjusting the composition of the edible IJ ink; however, unlike this, the present invention has achieved improvement in the light fastness of printed images by adjusting the printing method of the edible IJ ink itself. Thus, the present invention has a significantly different design idea from that of the prior art.

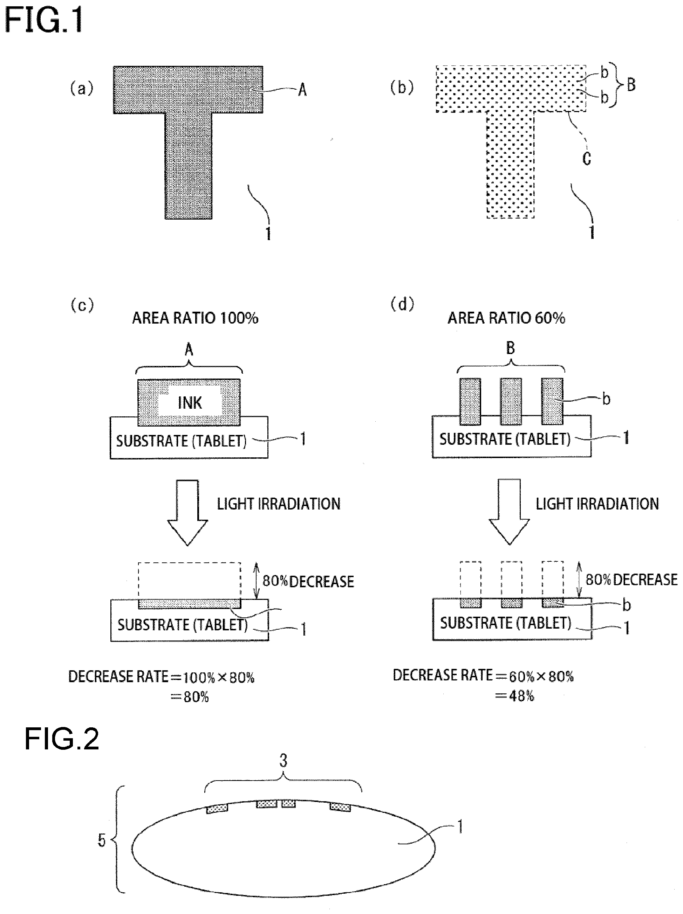

The edible IJ ink printing method according to the present embodiment and the reason why the light fastness of the printed image is improved by the printing method will be described in detail below with reference to Fig. 1.

Fig. 1 is a schematic diagram for explaining an example of the edible IJ ink printing method according to the present embodiment. Fig. 1 (a) is a plan view for explaining the form of a printed part printed without performing thinning printing (so-called "solid printed part"), which has generally been used in the prior art, and Fig. 1 (c) is a cross-sectional view for explaining the decrease rate of the print density in the solid printed part. Further, Fig. 1 (b) is a plan view for explaining the form of a printed part composed of a plurality of dots or halftone dots printed by thinning printing of the present embodiment (hereafter also referred to as "dot printed part" for convenience), and Fig. 1 (d) is a cross-sectional view for explaining the decrease rate of the print density in the dot printed part. That is, the dot printed part in the present embodiment refers to a region printed as dots or halftone dots by thinning print images or characters, which is different from the solid printed part formed by solid printing.

In the present embodiment, as shown in Fig. 1 (a), the area of a solid printed part A (the area of the entire region filled with the edible IJ ink) is defined as an "area ratio of 100%." Next, it is assumed that the when the solid printed part A is irradiated with light, the colorant decrease rate (light discoloration rate and light fading rate) in the entire solid printed part A is, for example, 80%. In that case, as shown in Fig. 1 (c), the "decrease rate of the print density" in the entire solid printed part A will be 80%. Here, the "decrease rate of the print density" is an index (parameter) defined by "area of printed part" × "colorant decrease rate." A larger value of the decrease rate of the print density indicates a higher degree of discoloration and fading caused by light irradiation.

On the other hand, Fig. 1 (b) illustrates a case where thinning printing is performed so that the area of a dot printed part B composed of a plurality of dots b (the total area of the dots b filled with the edible IJ ink) satisfies an "area ratio of 60%." If the edible IJ ink used for printing is the same, the colorant decrease rate (light discoloration rate and light fading rate) is a constant value, independent of the printing method (i.e., whether the printing method is solid printing or dot printing). Therefore, when the dot printed part B printed with the same edible IJ ink as the edible IJ ink used for printing the solid printed part A is irradiated with light, the decrease rate of the print density in the entire dot printed part B is 48% (decrease rate of print density = "area 60% of the printed part" × "colorant decrease rate 80%").

Thus, even if the amount of colorant reduction is the same before and after fading, regarding the printing area ratio (100%) of a printed part by solid printing (solid printed part A), which has been generally used in the prior art, the printing area ratio of a printed part by thinning printing (dot printed part B) is reduced, whereby the "decrease rate of the print density," which affects visibility, can be reduced. As a result, "ΔE," which is an index indicating the degree of light fastness (discoloration and fading), can be reduced.

In the present embodiment, when the ratio of the total area of a plurality of printed parts (dots b) printed while performing thinning printing to the area of a printed part (solid printed part A) printed without performing thinning printing is defined as a printing area ratio (i.e., total area of dots b/area of solid printed part A), thinning printing may be performed so that the printing area ratio is within the range of 30% or more and 80% or less, and more preferably within the range of 30% or more and 60% or less.

Wen the printing area ratio is within the above numerical range, the "decrease rate of the print density," which affects visibility, can be sufficiently reduced while obtaining sufficient visibility, and "ΔE," which is an index indicating the degree of light fastness (discoloration and fading), can be sufficiently reduced.

The "printing area ratio" described above has the same meaning as the ratio of the total area of a plurality of printed parts (dots b) printed with the edible IJ ink to the area of a virtually provided printed region (total area of printed parts (dots b) printed with edible IJ ink/area of virtually provided printed region). The "area of a virtually provided printed region" refers to the area of the entire region shown in the broken line c in Fig. 1 (b). Moreover, "a plurality of printed parts printed with the edible IJ ink" refers to the dots b shown in Fig. 1 (b).

Further, the "printing area ratio" described above has the same meaning as the ratio of the total area of a plurality of printed parts (dots b) printed by thinning printing to the area of the entire region to be thinning printed (total area of printed parts (dots b) printed by thinning printing/area of entire region to be thinning printed). The "area of the entire region to be thinning printed" means the area of the entire region shown in the broken line c in Fig. 1 (b). Further, "a plurality of printed parts printed by thinning printing" refers to the dots b shown in Fig. 1 (b).

In the present embodiment, each printed part (dot b) printed with the edible IJ ink may be circular in plan view, and the diameter of the circular shape may be within the range of 17 μm or more and 170 μm or less. Further, the diameter of the circular shape is more preferably within the range of 20 μm or more and 90 μm or less, and even more preferably within the range of 30 μm or more and 60 μm or less.

If the size of each printed part (dot b) printed with the edible IJ ink is within the above numerical range, the "decrease rate of the print density," which affects visibility, can be sufficiently reduced while obtaining sufficient visibility, and "ΔE", which is an index indicating the degree of light fastness (discoloration and fading), can be sufficiently reduced.

In the present embodiment, the optical density (OD value) of an image printed by the printing method described above is preferably 0.3 or more, and more preferably 0.3 or more and 0.42 or less.

If the optical density (OD value) of the printed image is the above numerical value, the "decrease rate of the print density," which affects visibility, can be sufficiently reduced while obtaining sufficient visibility, and "ΔE," which is an index indicating the degree of light fastness (discoloration and fading), can be sufficiently reduced.

The upper limit value of the optical density (OD value) of the printed image is not particularly limited; however, in consideration of the intended use, it is sufficient that the optical density (OD value) of the printed image is 1.0 or less.

As described above, the gist of the present invention is to focus on the numerical range of the "printing area ratio," which has not been taken into consideration in the prior art, and to set its numerical range to 30% or more and 80% or less, thereby improving the light fastness of printed images. Accordingly, thinning printing explained in the present embodiment is merely one means, and there is no particular limitation on the thinning printing process itself. For example, a plurality of printed parts (dots b) may be printed all at once or in several batches. From the viewpoint of preventing the bleeding of the printed parts (dots b), the printed parts (dots b) may be printed in two batches.

[Printing device]

The edible IJ ink according to the present embodiment is not particularly limited in terms of printing means (device), and can be printed using an inkjet device such as a commercially available inkjet printer. Therefore, the edible IJ ink according to the present embodiment has a wide range of applications and is very useful. For example, the edible IJ ink according to the present embodiment may be used for printing using a drop-on-demand inkjet device, which is actuated by piezoelectric elements (piezoelectric ceramic), or other inkjet devices.

Examples of drop-on-demand inkjet devices include a thermal-inkj et type device ejecting an IJ ink using water vapor pressure generated by instantaneously heating a micro-heating element to a high temperature (of 200°C to 300°C), an electrostatic-type device ejecting an IJ ink by electrostatically vibrating an actuator, and an ultrasonic-type device using an ultrasonic cavitation phenomenon. If the edible IJ ink according to the present embodiment is electrically chargeable, a continuously injectable (continuous type) device can also be used.

[Composition of edible IJ ink]

The edible IJ ink according to the present embodiment is, for example, an IJ ink containing an edible dye, wherein the edible dye is any one of red No. 2, red No. 3, red No. 40, red No. 102, red No. 104, red No. 105, red No. 106, yellow No. 4, yellow No. 5, green No. 3, blue No. 1, blue No. 2, and sodium copper chlorophyllin. With this composition, the light discoloration and light fading of printed images can be sufficiently prevented, and the light fastness of the edible IJ ink can be improved.

Each component that forms the edible IJ ink according to the present embodiment will be described below.

(Coloring material)

The colorant (coloring material) that can be added to the edible IJ ink according to the present embodiment is appropriately selected from, for example, conventionally known synthetic edible colorants and natural edible colorants.

Examples of the synthetic edible colorants include tar colorants, natural colorant derivatives, and natural synthetic colorants. Examples of the tar colorants include edible red No. 2 (Amaranth, FDA Name: FD & C Red No. 2, Color Index Name: Acid Red 27, CAS Number: 915-67-3), edible red No. 3 (FDA Name: FD & C Red No.3, Color Index Name: Acid Red 51, CAS Number: 16423-68-0), edible red No. 40 (Allura Red AC, FDA Name: FD & C Red No. 40, Color Index Name: Food Red 40, CAS Number: 25956-17-6), edible red No. 102 (New Coccine, Color Index Name: Acid Red 18, CAS Number: 2611-82-7), edible red No. 104 (Phloxine, FDA Name: D & C Red No. 28, Color Index Name: Acid Red 92, CAS Number: 18472-87-2), edible red No. 105 (Rose bengal, Color Index Name: Acid Red 94, CAS Number: 632-69-9), edible red No. 106 (Acid Red, Color Index Name: Acid Red 52, CAS Number: 3520-42-1), edible yellow No. 4 (Tartrazine, FDA Name: FD & C Yellow No. 5, Color Index Name: Acid Yellow 23, CAS Number: 1934-21-0), edible yellow No. 5 (Sunset Yellow FCF, FDA Name: FD & C Yellow No. 6, Color Index Name: Food Yellow 3, CAS Number: 2783-94-0), edible green No. 3 (Fast Green FCF, FDA Name: FD & C Green No. 3, Color Index Name: Food Green, CAS Number: 2353-45-9), edible blue No. 1 (Brilliant Blue FCF, FDA Name: FD & C Blue No. 1, Color Index Name: Food Blue 2, CAS Number: 3844-45-9), edible blue No. 2 (Indigo Carmine, FDA Name: FD & C Blue No. 2, Color Index Name: Acid Blue 74, CAS Number: 860-22-0), edible red No. 2 aluminum lake (FD & C Red No. 2 Aluminum Lake), edible red No. 3 aluminum lake (FD & C Red No. 3 Aluminum Lake), edible red No. 40 aluminum lake (FD & C Red No. 40 Aluminum Lake), edible yellow No. 4 aluminum lake (FD & C Yellow No. 5 Aluminum Lake), edible No. 5 aluminum lake (FD & C Yellow No.6 Aluminum Lake), edible blue No. 1 aluminum lake (FD & C Blue No. 1 Aluminum Lake), edible blue No. 2 aluminum lake (FD & C Blue No. 2 Aluminum Lake), and the like.

Examples of the natural colorant derivatives include norbixin potassium and the like. Examples of the natural synthetic colorants include β-carotene, riboflavin, sodium copper chlorophyllin, and the like.

The "Color Index Name" mentioned above is established by the American Association of Textile Chemists and Colorists. Further, the "FDA Name" mentioned above is established by the U.S. Food and Drug Administration (FDA).

Each of the available colorants (substances) is identified using CAS Number in the present embodiment; however, the present invention is not limited thereto. As a matter of course, for example, colorants (substances) that may be used in the present embodiment include those colorants which have the same substance names as those of the colorants (substances) described in the present embodiment but have different CAS Numbers because of being geometric isomers, stereoisomers, materials comprising isotopes, or salts thereof. If an isomer or the like is not present or an available colorant (substance) is specified (limited), the substance (compound) with CAS Number described in the present embodiment may be used.

Examples of the natural edible colorants include anthocyanin colorants, carotenoid colorants, quinone colorants, chlorophyll colorants, flavonoid colorants, betaine colorants, Monascus colorants, and other natural colorants originating from natural products. Examples of the anthocyanin colorants include red radish colorant, red cabbage colorant, red rice colorant, elderberry colorant, cowberry colorant, gooseberry colorant, cranberry colorant, salmon berry colorant, perilla colorant, sim blueberry colorant, strawberry colorant, dark sweet cherry colorant, cherry colorant, hibiscus colorant, huckleberry colorant, grape juice colorant, grape skin colorant, black currant colorant, blackberry colorant, blueberry colorant, plum colorant, whortleberry colorant, boysenberry colorant, mulberry colorant, purple yam colorant, purple corn colorant, Chinese purple yam colorant, raspberry colorant, red currant colorant, loganberry colorant, and other anthocyanin colorants. Examples of the carotenoid colorants include annatto colorant, gardenia yellow colorant, and other carotenoid colorants. Examples of the quinone colorants include cochineal colorant, Lithospermi radix colorant, lac colorant, and other quinone colorants. Examples of the flavonoid colorants include safflower yellow colorant, kaoliang colorant, onion colorant, and other flavonoid colorants. Examples of the betaine colorants include beet red colorant. Examples of the Monascus colorants include Monascus red colorant and Monascus yellow colorant. Examples of other colorants originating from natural products include turmeric colorant, Clerodendrum trichotomum colorant, gardenia red colorant, spirulina blue colorant, and the like.

(Discoloration inhibitor)

The edible IJ ink according to the present embodiment may contain, for example, a hydroxy acid salt having two or more carboxyl groups. The hydroxy acid salt functions as a discoloration inhibitor for red No. 3 and acts to inhibit the photolysis of red No. 3. Due to this, while the edible IJ ink containing red No. 3 maintains the initial print color (color immediately after printing), the light discoloration and light fading of the printed image can be sufficiently prevented, and light fastness can be improved. Specifically, the edible IJ ink containing red No. 3 may contain at least one of citrate or malate as the hydroxy acid salt having two or more carboxyl groups. The edible IJ ink containing red No. 3 may contain at least one of citrate or malate, or may contain both of citrate and malate.

The mixing ratio of the hydroxy acid salt (citrate and/or malate) in the edible IJ ink according to the present embodiment, i.e., the total content of the hydroxy acid salt, is preferably within the range of 0.3 mass% or more and 30 mass% or less based on the total mass of the ink. With such a configuration, the effect of the hydroxy acid salt as a discoloration inhibitor is more reliably exhibited, the light fastness of the edible IJ ink containing red No. 3 is reliably improved, and the hydroxy acid salt can be prevented from remaining undissolved during production of the edible IJ ink. For example, if no residual hydroxy acid salt is visually observed after performing a step of stirring a solvent and the hydroxy acid salt during production of the edible IJ ink (e.g., a step of adding the hydroxy acid salt to a solvent at 25°C, followed by stirring for 60 minutes), it is deemed that the solubility of the hydroxy acid salt in the solvent is ensured.

In contrast, if the mixing ratio of the hydroxy acid salt is less than 0.3 mass%, the effect of the hydroxy acid salt as an ink discoloration inhibitor may be reduced. If the mixing ratio of the hydroxy acid salt exceeds 30 mass%, part of the hydroxy acid salt may remain undissolved in the stirring step during production of the edible IJ ink.

(Citrate)

Among the above hydroxy acid salts that can be contained as a discoloration inhibitor in the edible IJ ink according to the present embodiment, examples of the citrate include isopropyl citrate, triethyl citrate, monopotassium citrate, tripotassium citrate, calcium citrate, sodium ferrous citrate, ferrous citrate, ammonium ferric citrate, sodium dihydrogen citrate, disodium citrate, trisodium citrate, and the like.

(Malate)

Further, among the above hydroxy acid salts that can be contained as a discoloration inhibitor in the edible IJ ink according to the present embodiment, examples of the malate include sodium malate. Examples of sodium malates include sodium ferrous malate, disodium malate, and the like. In addition to these, other usable examples as discoloration inhibitors include isopropyl malate, diethyl malate, monopotassium malate, dipotassium malate, calcium malate, iron malate, ammonium iron malate, and the like.

The edible IJ ink according to the present embodiment may contain, as a discoloration inhibitor that also acts as a fixing agent, for example, a disaccharide whose solubility in 100 ml of water at 20°C is 39 g or less. Specifically, lactose and cellobiose can fix red No. 102 and blue No. 1 to the surface of a material to be printed. Moreover, reduced isomaltulose can fix blue No. 1 to the surface of a material to be printed. In 100 ml of water at 20°C, the solubility of lactose is 16 g, the solubility of cellobiose is 14 g, and the solubility of reduced isomaltulose is 38 g. When the discoloration inhibitor has such a configuration, the ink containing the specific tar colorant can be fixed to the surface of a material to be printed. This can sufficiently prevent the light discoloration and fading of the printed image and improve the light fastness. Further, the above disaccharides (lactose, cellobiose, and reduced isomaltulose) used as discoloration inhibitors in the present embodiment also have the function of suppressing the decomposition of the inkjet ink due to light irradiation (photolysis). Therefore, the occurrence of light discoloration and fading itself can be prevented.

The mixing ratio of the discoloration inhibitor in the inkjet ink according to the present embodiment, i.e., the total content of the disaccharides (lactose, cellobiose, and reduced isomaltulose), is preferably within the range of 0.5 mass% or more and 20 mass% or less based on the total mass of the ink. With this configuration, the ink fixation effect of the disaccharides is more reliably exhibited, and the light fastness of the inkjet ink can be reliably improved. Further, this can impart high intermittent resumability to the inkjet ink according to the present embodiment.

In contrast, if the mixing ratio of the discoloration inhibitor is less than 0.5 mass%, the ink fixation effect may be reduced. If the mixing ratio of the discoloration inhibitor (lactose, cellobiose, or reduced isomaltulose) exceeds 20 mass%, the discoloration inhibitor in the ink may precipitate or deposit as a solid due to an increase in ink viscosity or a deterioration in the dissolution stability of the discoloration inhibitor, which can cause nozzle clogging in the inkjet head during printing and reduce intermittent resumability.

(Solvent)

The edible IJ ink according to the present embodiment may contain a solvent (dispersion medium) to dissolve (disperse) the above described coloring materials etc. The edible IJ ink according to the present embodiment may contain at least one of water (e.g., purified water) and alcohol as the solvent. The alcohol may contain, for example, at least one of ethanol, NPA, and IPA. Because they are highly volatile, the transfer resistance (dryness) of the edible IJ ink can be improved. By adding these components to the solvent in addition to water, the performance of each component can be imparted to the edible IJ ink.

When the solvent contains alcohol such as ethanol, the mixing ratio of the alcohol, i.e., the amount of alcohol added, may be within the range of 6.5 mass% or more and 55 mass% or less based on the total mass of the ink. With such a configuration, the transfer resistance (dryness) of the edible IJ ink can be improved.

Further, a solvent other than those described above may be added to the edible IJ ink according to the present embodiment. Examples of the solvent that can be added to the edible IJ ink according to the present embodiment include propylene glycol, glycerin, polyethylene glycol 300 (having an average molecular weight of 300), ethyl lactate, and the like. The mixing ratio is not particularly limited; however, the ink may have a content of any of propylene glycol, glycerin, and polyethylene glycol 300 within the range of 0.1 mass% or more and 40 mass% or less to prevent the ink from drying at the nozzle.

(Light fastness)

The details of light fastness in an image printed by using the edible IJ ink printing method according to the present embodiment will be described below.

When a light fastness test is performed on a printed image generated by using the edible IJ ink printing method according to the present embodiment (i.e., a printed image with an adjusted printing area ratio), the reduction rate in the color difference ΔE before and after the light fastness test in JIS Z 8781 compared with ΔE in solid printing is preferably 20% or more, and more preferably 40% or more. Solid printing refers to printing images or characters without thinning out, and the light fastness test refers to a test that compares the color difference ΔE (according to JIS Z 8781), which indicates variation in chromaticity and optical color density before and after visible light irradiation, in an image printed by using, for example, the edible IJ ink printing method according to the present embodiment. Specifically, the color difference ΔE is measured before and after irradiating the printed image with cumulative visible light of 1.2 million lux, and the values are compared. For example, a xenon weather meter (Ci4000, Toyo Seiki Seisaku-sho, Ltd.) is used for irradiation with visible light.

In the printing method according to the present embodiment, printing is performed so that the printing area ratio is within the range of 30% or more and 80% or less, which can keep the decrease rate of the print density in the tablet surface low. Accordingly, the light discoloration and light fading of the printed image can be sufficiently prevented. This can improve the light fastness of the image printed using the edible IJ ink.

(OD value: Evaluation of discoloration of printed matter)

The details of the OD value measurement in an image printed by using the edible IJ ink printing method according to the present embodiment will be described below.

The purified ink was used to print a circular image (4.0 mm in diameter) with a bending mode piezo inkjet head printer on a test uncoated tablet (based on starch) and a film-coated (FC) tablet (based on adjusted starch, coated with a mixture of 70% hydroxypropyl methylcellulose and 30% titanium oxide, 6.5 mm in diameter) so that the printing area ratio was within the range of 30% or more and 80% or less.

A spectrometer "X-Rite 530," manufactured by X-RITE Inc., was placed above the printed images of the printed uncoated tablet and the printed film-coated tablet to measure the color tone (L*a*b* color system) and OD value. The setting conditions for the above measurement are a viewing angle of 2 degrees and a D50 light source.

In this way, the optical density (OD value) of the printed image according to the present embodiment was measured.

(Internal-sizing resin)

The edible IJ ink according to the present embodiment may contain an internal-sizing resin in addition to the above colorant and solvent. The internal-sizing resin that can be added to the edible IJ ink according to the present embodiment may be an edible resin-like material in the form of a water-soluble powder, paste or flakes which are capable of forming a coating on the surface of a tablet when dried after printing. Examples of the internal-sizing resin include polyvinyl alcohol (PVA), hydroxypropyl cellulose (HPC), hydroxypropyl methylcellulose (HPMC), polyvinylpyrrolidone (PVP), high molecular weight polyethylene glycol (PEG) such as polyethylene glycol 4000 or polyethylene glycol 1540, shellac resin, methacrylic acid copolymer (product name: Eudragit S 100), maltodextrin, erythritol, and the like.

(Leveling agent)

The edible IJ ink according to the present embodiment may contain a leveling agent in addition to the above colorant, solvent, or internal-sizing resin. The levelling agent that can be added to the edible IJ ink according to the present embodiment may be an edible and water-soluble surfactant. Examples of the levelling agent include polyglycerin fatty acid ester (e.g., Decaglyceryl distearate Q-182S or Decaglyceryl monolaurate Q-12S manufactured by Taiyo Kagaku Co., Ltd.), sorbitan fatty acid ester (e.g., NIKKOLSL-10 manufactured by Nikko Chemicals Co., Ltd.), sucrose fatty acid ester (e.g., DK Ester F-110 manufactured by DKS Co. Ltd.), polysorbate (Emazole S-120 series manufactured by Kao Corporation), and the like.

(Discoloration inhibitor)

The edible IJ ink according to the present embodiment may contain a discoloration inhibitor in addition to the above colorant, solvent, internal-sizing resin, or leveling agent. The discoloration inhibitor that can be added to the edible IJ ink according to the present embodiment may be edible. Examples of the discoloration inhibitor include reduced isomaltulose, citrate, malate, lactose, cellobiose, and the like. The mixing ratio of the discoloration inhibitor, i.e., the amount of the discoloration inhibitor added, is preferably within the range of 0.3 mass% or more and 20 mass% or less based on the total mass of the ink. This configuration can improve the deterioration of the edible IJ ink due to oxidation.

[Tablet]

The edible IJ ink according to the present embodiment may be used for printing characters or images on, for example, the surface of a tablet, using the printing method described above. That is, the tablet according to the present embodiment may have a printed part printed using the edible IJ ink printing method according to the present embodiment, i.e., a printed image that is thinning printed so that the printing area ratio is within the range of 30% or more and 80% or less. The edible IJ ink printing method according to the present embodiment can improve the light fastness of a printed image (e.g., printed image 3), for example, printed on the surface of a pharmaceutical tablet using an inkjet printing method. The following describes the configuration of a tablet comprising a printed image obtained by thinning printing using the edible IJ ink printing method according to the embodiment so that the printing area ratio was within the range of 30% or more and 80% or less.

The tablet according to the present embodiment may be, for example, a pharmaceutical tablet. Examples of the "pharmaceutical tablet" include a film-coated tablet having an outermost surface on which a water-soluble surface layer is formed, as well as an uncoated tablet (bare tablet), a sugar-coated tablet, an enteric tablet, and an orally disintegrating tablet.

Fig. 2 is a schematic cross-sectional view illustrating an example of a pharmaceutical tablet (uncoated tablet) having a print pattern (characters or image). Fig. 2 shows a cross section of a printed uncoated tablet 5 having a printed image 3, such as characters, printed on a top face of a tablet substrate 1. The "printed image 3" shows a printed image obtained by thinning printing using the edible IJ ink printing method according to the embodiment so that the printing area ratio was within the range of 30% or more and 80% or less. In addition, the "printed image 3" described later also shows a printed image obtained by thinning printing.

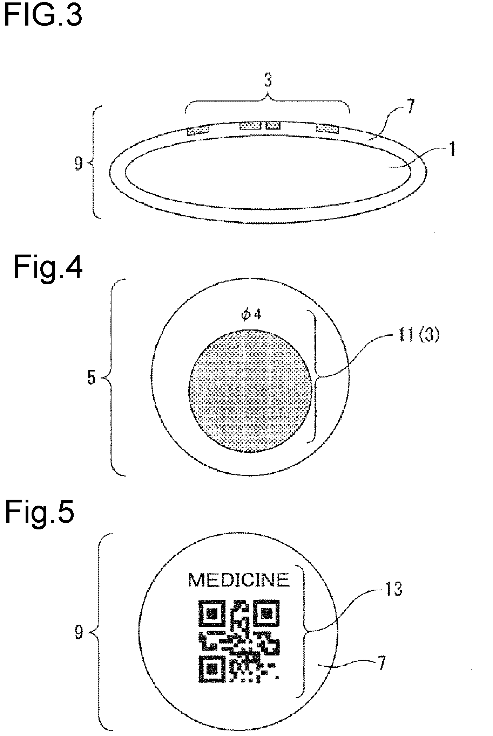

Fig. 3 is a schematic cross-sectional view illustrating an example of a pharmaceutical tablet (film-coated (FC) tablet) having a print pattern (characters or image). Fig. 3 shows a cross section of a printed film-coated tablet 9 in which a printed image 3, such as characters, is printed on a top face of a tablet substrate 1 having a film-coated layer 7 formed on a surface thereof.

In the present embodiment, a printed image 3 may be printed as an uncoated tablet printed image 11 as shown in Fig. 4, or a two-dimensional barcode may be printed as a film-coated tablet printed image 13 as shown in Fig. 5.

There is no particular limitation on the active ingredients contained in the pharmaceutical tablet. Examples of the active ingredients include a substance effective for preventing or treating various diseases (e.g., a substance having sleep-inducing effect, an tranquilizer activity, an antibacterial activity, an antihypertensive effect, an anti-angina activity, an analgesic effect, an anti-inflammatory activity, a tranquilizing effect, a diabetes treatment activity, a diuretic effect, an anticholinergic activity, an anti-hyperacidity effect, an antiepileptic effect, an ACE inhibitory activity, a β-receptor antagonist or agonist activity, an anesthetic action, an appetite suppressant action, an antiarrhythmic effect, an antidepressant effect, an anticoagulant activity, an antidiarrheal effect, an antihistamine activity, an antimalarial effect, an antitumor activity, an immunosuppressive activity, an antiparkinsonian effect, an antipsychotic effect, an antiplatelet activity, an antihyperlipidemic effect, and the like), a substance having a scavenging effect, and a substance having a scent or a deodorant action, but are not limited thereto.

The tablet according to the present embodiment may as necessary contain a carrier that is acceptable for its application, together with an active ingredient. For example, a pharmaceutical tablet may comprise a pharmaceutically acceptable carrier. As the pharmaceutically acceptable carrier, various organic or inorganic carrier materials may be used as a pharmaceutical material, and an appropriate amount of excipients, lubricants, binders, disintegrating agents, thickeners, or the like may be mixed thereto as appropriate. As necessary, additives, such as a preservative, an antioxidant, a coloring agent, and a sweetening agent, may also be used.

Although the present embodiment has been described taking an example of a pharmaceutical tablet as a tablet, the tablet is not limited to the tablet of the present invention. There is no particular limitation on the object to be printed by the edible IJ ink printing method according to the present embodiment. The ink may be printed on the surface of various tablets, such as a tablet to be administered to animals other than humans (e.g., pets, domestic animals, poultry), a feed tablet, a fertilizer tablet, a cleaning agent tablet, a tablet sweet such as a lemonade tablet, a supplement tablet, or the like. The edible IJ ink printing method according to the present embodiment does not particularly limit the size of the object to be printed, but may be applied to tablets of various sizes.

(Effects of the present embodiment)

(1) In the tablet according to the present embodiment, when a ratio of the total area of a plurality of dots b printed while performing thinning printing to the area of a solid printed part A printed without performing thinning printing (total area of dots b printed while performing thinning printing/area of solid printed part A printed without performing thinning printing) is defined as a printing area ratio, the printing area ratio is within the range of 30% or more and 80% or less, an ink used for printing is an edible IJ ink, and the edible IJ ink comprises an edible dye.
With such a configuration, compared with the prior art, light discoloration and light fading of the printed image 3 directly printed on the tablet surface etc. can be sufficiently prevented, and the light fastness of the printed image 3 can be sufficiently improved. Further, the printed image portion on the surface of the tablet can be imparted with edibility.
(2) The tablet according to the present embodiment may comprise, as an edible dye, any one of red No. 2, red No. 3, red No. 40, red No. 102, red No. 104, red No. 105, red No. 106, yellow No. 4, yellow No. 5, green No. 3, blue No. 1, blue No. 2, and sodium copper chlorophyllin. With such a configuration, compared with the prior art, light discoloration and light fading of the printed image 3 directly printed on the tablet surface etc. can be sufficiently prevented, and the light fastness of the printed image 3 can be sufficiently improved. Further, the printed image portion on the surface of the tablet can be imparted with edibility.
(3) The tablet according to the present embodiment may be a film-coated tablet.
With such a configuration, compared with the prior art, the light fastness of the printed image can be further improved.
(4) In the tablet according to the present embodiment, the printed image may have an optical density (OD value) of 0.3 or more.

With such a configuration, compared with the prior art, the visibility can be improved while improving the light fastness of the printed image.

(5) In the tablet according to the present embodiment, the edible ink may be an inkjet ink.

With such a configuration, inkjet printing is possible.

(6) In the printed matter according to the present embodiment, when a ratio of the total area of a plurality of dots b printed while performing thinning printing to the area of a solid printed part A printed without performing thinning printing (total area of dots b printed while performing thinning printing/area of solid printed part A printed without performing thinning printing) is defined as a printing area ratio, the printing area ratio is within the range of 30% or more and 80% or less, an ink used for printing is an edible IJ ink, and the edible IJ ink comprises an edible dye.

With such a configuration, compared with the prior art, the light discoloration and light fading of the printed image 3 directly printed on the surface of the printed matter etc. can be sufficiently prevented, and the light fastness of the printed image 3 can be sufficiently improved. Further, the printed image portion on the surface of the printed matter can be imparted with edibility.

(7) The printed matter according to the present embodiment may comprise, as an edible dye, any one of red No. 2, red No. 3, red No. 40, red No. 102, red No. 104, red No. 105, red No. 106, yellow No. 4, yellow No. 5, green No. 3, blue No. 1, blue No. 2, and sodium copper chlorophyllin. With such a configuration, compared with the prior art, the light discoloration and light fading of the printed image 3 directly printed on the surface of the printed matter etc. can be sufficiently prevented, and the light fastness of the printed image 3 can be sufficiently improved. Further, the printed image portion on the surface of the printed matter can be imparted with edibility.

(8) In the printed matter according to the present embodiment, the edible ink may be an inkjet ink.

With such a configuration, inkjet printing is possible.

(9) The edible IJ ink printing method according to the present embodiment comprises performing thinning printing so that when a ratio of the total area of a plurality of dots b printed while performing thinning printing to the area of a solid printed part A printed without performing thinning printing (total area of dots b printed while performing thinning printing/area of solid printed part A printed without performing thinning printing) is defined as a printing area ratio, the printing area ratio is within the range of 30% or more and 80% or less, an ink used for printing being an edible IJ ink, and the edible IJ ink comprising an edible dye.

With such a configuration, compared with the prior art, light discoloration and light fading of the printed image 3 directly printed on the tablet surface etc. can be sufficiently prevented, and the light fastness of the printed image 3 can be sufficiently improved. Further, the printed image portion on the surface of the tablet can be imparted with edibility.

(10) In the edible IJ ink printing method according to the present embodiment, the edible dye may comprise any one of red No. 2, red No. 3, red No. 40, red No. 102, red No. 104, red No. 105, red No. 106, yellow No. 4, yellow No. 5, green No. 3, blue No. 1, blue No. 2, and sodium copper chlorophyllin. With such a configuration, compared with the prior art, light discoloration and light fading of the printed image 3 directly printed on the tablet surface etc. can be sufficiently prevented, and the light fastness of the printed image 3 can be sufficiently improved. Further, the printed image portion on the surface of the tablet can be imparted with edibility.

(11) In the edible IJ ink printing method according to the present embodiment, the edible ink may further contain alcohol as a solvent.

With such a configuration, compared with the prior art, the ink can be imparted with sufficient dryness.

(12) In the edible IJ ink printing method according to the present embodiment, the alcohol may contain at least one of ethanol, NPA, and IPA.

With such a configuration, compared with the prior art, the ink can be imparted with sufficient dryness.

(13) In the edible IJ ink printing method according to the present embodiment, the amount of alcohol added may be 6.5 mass% or more based on the total mass of the edible ink.

With such a configuration, compared with the prior art, the ink can be imparted with sufficient dryness.

(14) In the edible IJ ink printing method according to the present embodiment, the edible ink may further contain a discoloration inhibitor.

With such a configuration, compared with the prior art, the ink can be imparted with sufficient light fastness.

(15) In the edible IJ ink printing method according to the present embodiment, the content of the discoloration inhibitor may be within the range of 0.3 mass% or more and 20 mass% or less based on the total mass of the edible ink.

With such a configuration, compared with the prior art, the ink can be imparted with sufficient light fastness.

(16) In the edible IJ ink printing method according to the present embodiment, the discoloration inhibitor may be reduced isomaltulose, citrate, malate, lactose, or cellobiose.

With such a configuration, compared with the prior art, the ink can be imparted with sufficient light fastness.

(17) In the edible IJ ink printing method according to the present embodiment, each printed part printed with the edible ink has a circular shape in plan view, and the diameter of the circular shape may be within the range of 17 $\mu$m or more and 170 $\mu$m or less.

With such a configuration, compared with the prior art, light discoloration and light fading of the printed image 3 directly printed on the tablet surface etc. can be sufficiently prevented, and the light fastness of the printed image 3 can be sufficiently improved.

(18) Printing may be done on tablets using the edible IJ ink printing method according to the present embodiment. With such a configuration, compared with the prior art, the light discoloration and light fading of the printed image 3 directly printed on the tablet surface etc. can be sufficiently prevented, and the light fastness of the printed image 3 can be sufficiently improved.

(19) Further, in the edible IJ ink printing method according to the present embodiment, the edible ink may be an inkjet ink.

With such a configuration, inkjet printing is possible.

[Examples]

The present invention will be further described in detail by way of examples; however, the present invention is not limited to these examples.

(Production of inkjet ink)

First, a printing ink was prepared. The inkjet ink contains components of a colorant and a solvent (an organic solvent and water), and may also contain a discoloration inhibitor. The first step of the preparation was to mix water with an organic solvent to produce a combined solution (solvent). Next, when a discoloration inhibitor was contained, the discoloration inhibitor was added to the combined solution to produce a transparent base liquid. Finally, a colorant was added to the combined solution or the transparent base liquid. In this way, the ink according to the present example was prepared. Each component will be specifically described below.

In the present example, purified water (ion-exchanged water) was used as water to compose the ink. Further, ethanol and propanol (NPA and IPA) were each used as the organic solvent. Purified water, ethanol, NPA, and IPA were added in the amounts shown in Tables 1 and 2, and stirred well to produce a mixed solvent. As a discoloration inhibitor, reduced isomaltulose, tripotassium citrate, or lactose was added in the amount shown in Tables 1 and 2 to the mixed solvent, followed by stirring for about 1 hour to obtain a transparent base liquid.

A different amount of colorant was added to the transparent base liquid, thereby obtaining inks of Examples 1 to 25 and inks of Comparative Examples 1 to 18 (each 100 g). The composition of each ink is shown in Tables 1 and 2.

[Table 1]

| | | | Comp. Ex. 1 | Ex. 1 | Comp. Ex. 2 | Ex. 2 | Ex. 3 | Comp. Ex. 3 | Ex. 4 | Ex. 5 | Comp. Ex. 4 | Ex. 6 | Ex. 7 | Comp. Ex. 5 | Ex. 8 | Ex. 9 | Comp. Ex. 6 | Ex. 10 | Comp. Ex. 7 | Ex. 11 | Ex. 12 | Comp. Ex. 8 | Ex. 13 | Comp. Ex. 9 | Ex. 14 |
|---|---|---|---|---|---|---|---|---|---|---|---|---|---|---|---|---|---|---|---|---|---|---|---|---|---|
| Composition | Colorant | Total amount | 4.0% | 4.0% | 4.0% | 4.0% | 4.0% | 3.2% | 3.2% | 3.2% | 2.4% | 2.4% | 2.4% | 5.2% | 5.2% | 5.2% | 4.0% | 4.0% | 4.0% | 4.0% | 4.0% | 3.2% | 3.2% | 3.2% | 3.2% |
| | | Red 2 | | | | | | | | | | | | | | | | | | | | | | | |
| | | Red 3 | | | | | | | | | | | | | | | 2.9% | 2.9% | 2.9% | 2.9% | 2.9% | | | | |
| | | Red 40 | | | | | | | | | | | | | | | | | | | | | | | |
| | | Red 102 | 1.0% | 1.0% | 1.0% | 1.0% | 1.0% | 0.8% | 0.8% | 0.8% | 0.6% | 0.6% | 0.6% | 0.8% | 0.8% | 0.8% | | | | | | | | 0.8% | 0.8% |
| | | Red 104 | | | | | | | | | | | | 1.2% | 1.2% | 1.2% | | | | | | | | | |
| | | Red 105 | | | | | | | | | | | | | | | 1.0% | 1.0% | 1.0% | 1.0% | 1.0% | | | | |
| | | Red 106 | | | | | | | | | | | | | | | | | | | | 1.0% | 1.0% | | |
| | | Yellow 4 | 2.0% | 2.0% | 2.0% | 2.0% | 2.0% | 1.6% | 1.6% | 1.6% | 1.2% | 1.2% | 1.2% | | | | | | | | | 1.4% | 1.4% | 1.6% | 1.6% |
| | | Yellow 5 | | | | | | | | | | | | | | | | | | | | | | | |
| | | Green 3 | | | | | | | | | | | | | | | | | | | | | | | |
| | | Blue 1 | 1.0% | 1.0% | 1.0% | 1.0% | 1.0% | 0.8% | 0.8% | 0.8% | 0.6% | 0.6% | 0.6% | 3.2% | 3.2% | 3.2% | 0.1% | 0.1% | 0.1% | 0.1% | 0.1% | 0.8% | 0.8% | 0.8% | 0.8% |
| | | Blue 2 | | | | | | | | | | | | | | | | | | | | | | | |
| | | Copper chlorophyllin Na | | | | | | | | | | | | | | | | | | | | | | | |
| | Solvent | Water | 66% | 66% | 62% | 62% | 62% | 62% | 62% | 62% | 64% | 64% | 64% | 68% | 68% | 68% | 56% | 56% | 56% | 56% | 56% | 62% | 62% | 62% | 62% |
| | | Ethanol | 30% | 30% | 30% | 30% | 30% | 30% | 30% | 30% | 30% | 30% | 30% | 6.5% | 6.5% | 6.5% | 40% | 40% | 40% | 40% | 40% | | | | |
| | | NPA | | | | | | | | | | | | | | | | | | | | 30% | 30% | | |
| | | IPA | | | | | | | | | | | | | | | | | | | | | | 30% | 30% |
| | Discoloration inhibitor | Total amount | 0.0% | 0.0% | 4.0% | 4.0% | 4.0% | 4.8% | 4.8% | 4.8% | 4.0% | 4.0% | 4.0% | 20% | 20% | 20% | 0.0% | 0.0% | 0.3% | 0.3% | 0.3% | 4.8% | 4.8% | 4.8% | 4.8% |
| | | Reduced isomaltulose | | | 4.0% | 4.0% | 4.0% | 4.8% | 4.8% | 4.8% | 4.0% | 4.0% | 4.0% | 20% | 20% | 20% | | | | | | 4.8% | 4.8% | 4.8% | 4.8% |

13

EP 4 501 319 A1

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 |
|---|---|---|---|---|---|---|---|---|---|---|---|---|---|---|---|---|---|---|---|---|---|---|---|
| Tripotassium citrate | | | | | | | | | | | | | | | | | 0.3% | 0.3% | 0.3% | | | | |
| Lactose | | | | | | | | | | | | | | | | | | | | | | | |
| Total | 100% | 100% | 100% | 100% | 100% | 100% | 100% | 100% | 100% | 100% | 100% | 100% | 100% | 100% | 100% | 100% | 100% | 100% | 100% | 100% | 100% | 100% | 100% |
| Printing area ratio | 100% | 35% | 100% | 50% | 35% | 100% | 60% | 40% | 100% | 80% | 50% | 100% | 80% | 30% | 100% | 60% | 100% | 80% | 60% | 100% | 50% | 100% | 50% |
| Print density OD value | 0.85 | 0.32 | 0.86 | 0.47 | 0.31 | 0.76 | 0.49 | 0.33 | 0.7 | 0.57 | 0.38 | 0.96 | 0.83 | 0.31 | 0.56 | 0.31 | 0.54 | 0.46 | 0.3 | 0.77 | 0.43 | 0.76 | 0.42 |
| Light fastness — ΔE | 42 | 25 | 32 | 24 | 17 | 22 | 16 | 12 | 18 | 14 | 9 | 20 | 16 | 12 | 29 | 17 | 23 | 17 | 13 | 23 | 14 | 23 | 13 |
| Light fastness — Comparison target | - | Comp. Ex. 1 | - | Comp. Ex. 2 | Comp. Ex. 2 | - | Comp. Ex. 3 | Comp. Ex. 3 | - | Comp. Ex. 4 | Comp. Ex. 4 | - | Comp. Ex. 5 | Comp. Ex. 5 | - | Comp. Ex. 6 | - | Comp. Ex. 7 | Comp. Ex. 7 | - | Comp. Ex. 8 | - | Comp. Ex. 9 |
| Light fastness — Reduction rate from comparison target ΔE | - | 40% | - | 25% | 47% | - | 27% | 45% | - | 22% | 50% | - | 20% | 40% | - | 41% | - | 27% | 43% | - | 39% | - | 43% |
| Light fastness — Determination | - | Good | - | Fair | Good | - | Fair | Good | - | Fair | Good | - | Fair | Good | - | Good | - | Fair | Good | - | Fair | - | Good |

14

[Table 2]

| | | | Comp. Ex. 10 | Ex. 15 | Comp. Ex. 11 | Ex. 16 | Comp. Ex. 12 | Ex. 17 | Ex. 18 | Ex. 19 | Comp. Ex. 13 | Ex. 20 | Comp. Ex. 14 | Ex. 21 | Comp. Ex. 15 | Ex. 22 | Comp. Ex. 16 | Ex. 23 | Comp. Ex. 17 | Ex. 24 | Comp. Ex. 18 | Ex. 25 |
|---|---|---|---|---|---|---|---|---|---|---|---|---|---|---|---|---|---|---|---|---|---|---|
| Composition | Colorant | Total amount | 4.2% | 4.2% | 4.2% | 4.2% | 4.2% | 4.2% | 4.2% | 4.2% | 3.0% | 3.0% | 5.0% | 5.0% | 4.2% | 4.2% | 1.0% | 1.0% | 6.0% | 6.0% | 6.0% | 6.0% |
| | | Red 2 | | | | | | | | | | | 0.3% | 0.3% | | | | | | | | |
| | | Red 3 | | | | | | | | | | | | | 3.0% | 3.0% | | | | | | |
| | | Red 40 | 0.2% | 0.2% | | | | | | | | | | | | | | | | | | |
| | | Red 102 | 2.2% | 2.2% | 3.7% | 3.7% | 3.7% | 3.7% | 3.7% | 3.7% | 1.5% | 1.5% | | | | | | | 6.0% | 6.0% | | |
| | | Red 104 | | | | | | | | | | | | | | | | | | | | |
| | | Red 105 | | | | | | | | | | | | | | | | | | | | |

EP 4 501 319 A1

15

EP 4 501 319 A1

| | | | | | | | | | | | | | | | | | | | | | |
|---|---|---|---|---|---|---|---|---|---|---|---|---|---|---|---|---|---|---|---|---|---|
| | Red 106 | | | | | | | | | | | | | | | | | | | | |
| | Yellow 4 | 0.6% | 0.6% | 0.2% | 0.2% | 0.2% | 0.2% | 0.2% | 0.2% | | | | | | | | | | | | |
| | Yellow 5 | | | | | | | | | 0.5% | 0.5% | 0.2% | 0.2% | | | | | | | | |
| | Green 3 | | | | | | | | | | | | | 1.1% | 1.1% | | | | | | |
| | Blue 1 | 1.2% | 1.2% | 0.2% | 0.2% | 0.2% | 0.2% | 0.2% | 0.2% | 1.0% | 1.0% | 0.5% | 0.5% | | | 1.0% | 1.0% | | | | |
| | Blue 2 | | | | | | | | | | | | | 0.1% | 0.1% | | | | | | |
| | Copper chlorophyllin Na | | | | | | | | | 4.0% | 4.0% | | | | | | | | | 6.0% | 6.0% |
| Solvent | Water | 62% | 62% | 66% | 66% | 64% | 64% | 64% | 64% | 42% | 42% | 85% | 85% | 85% | 85% | 63% | 63% | 63% | 63% | 88% | 88% |
| | Ethanol | 30% | 30% | 30% | 30% | 30% | 30% | 30% | 30% | 55% | 55% | 6.5% | 6.5% | 6.5% | 6.5% | 30.0% | 30.0% | 30.0% | 30.0% | 6.5% | 6.5% |
| | NPA | | | | | | | | | | | | | | | | | | | | |
| | IPA | | | | | | | | | | | | | | | | | | | | |
| Discoloration inhibitor | Total amount | 4.0% | 4.0% | 0.0% | 0.0% | 1.5% | 1.5% | 1.5% | 1.5% | 0.5% | 0.5% | 4.0% | 4.0% | 4.0% | 4.0% | 6.0% | 6.0% | 1.5% | 1.5% | 0.0% | 0.0% |
| | Reduced isomaltulose | 4.0% | 4.0% | | | | | | | | | 4.0% | 4.0% | | | 6.0% | 6.0% | | | | |
| | Tripotassium citrate | | | | | | | | | | | | | 4.0% | 4.0% | | | | | | |
| | Lactose | | | | | 1.5% | 1.5% | 1.5% | 1.5% | 0.5% | 0.5% | | | | | | | 1.5% | 1.5% | | |
| Total | | 100% | 100% | 100% | 100% | 100% | 100% | 100% | 100% | 100% | 100% | 100% | 100% | 100% | 100% | 100% | 100% | 100% | 100% | 100% | 100% |
| Printing area ratio | | 100% | 50% | 100% | 50% | 100% | 80% | 60% | 50% | 100% | 70% | 100% | 50% | 100% | 50% | 100% | 80% | 100% | 80% | 100% | 80% |
| Print density OD value | | 0.91 | 0.5 | 0.64 | 0.3 | 0.63 | 0.44 | 0.34 | 0.3 | 0.49 | 0.35 | 0.6 | 0.34 | 0.58 | 0.3 | 0.36 | 0.3 | 0.4 | 0.33 | 0.4 | 0.34 |
| Light fastness | ΔE | 27 | 16 | 28 | 16 | 22 | 17 | 15 | 13 | 21 | 15 | 18 | 9 | 19 | 12 | 22 | 17 | 20 | 16 | 14 | 11 |
| | Comparison target | - | Comp. Ex. 10 | - | Comp. Ex. 11 | - | Comp. Ex. 12 | Comp. Ex. 12 | Comp. Ex. 12 | - | Comp. Ex. 13 | - | Comp. Ex. 14 | - | Comp. Ex. 15 | - | Comp. Ex. 16 | - | Comp. Ex. 17 | - | Comp. Ex. 18 |

| Reduction rate from comparison on target ΔE | - | -39% | - | -43% | - | -25% | -34% | -41% | - | -29% | - | -50% | - | -37% | - | -23% | - | -20% | - | -21% |
|---|---|---|---|---|---|---|---|---|---|---|---|---|---|---|---|---|---|---|---|---|
| Determination | - | Fair | - | Good | - | Fair | Fair | Good | - | Fair | - | Good | - | Fair | - | Fair | - | Fair | - | Fair |

Solid foreign matter in the liquid was removed by passing 100 g of each of the above inks through a membrane filter. More specifically, each ink was passed through a membrane filter (cellulose acetate film) having a pore diameter of 5.0 μm once, followed by a membrane filter (cellulose acetate film) having a pore diameter of 0.8 μm once, to obtain purified ink, each of which weighed 99 g.

(Printing)

The purified ink was used to print a circular solid printed image (4.0 mm in diameter) with a bending mode piezo inkjet head printer on a test film-coated tablet (based on adjusted starch, coated with a mixture of 70% hydroxypropyl methylcellulose and 30% titanium oxide). In this way, film-coated tablets printed in each color were obtained. In the present example, the printing area ratio of the solid printed image (solid printed part) is defined as "100%."

The purified ink was used to print a circular thinning printed image (4.0 mm in diameter) with an adjusted printing area ratio with a bending mode piezo inkjet head printer on a test film-coated tablet (based on adjusted starch, coated with a mixture of 70% hydroxypropyl methylcellulose and 30% titanium oxide). In this way, film-coated tablets printed in each color were obtained. The printing area ratio of the thinning printed image (dot printed part) is as shown in Tables 1 and 2.

(Light fastness test)

A spectrometer "X-Rite 530," manufactured by X-RITE Inc., was placed above the solid printed image or thinning printed image of the printed film-coated tablet to measure the color tone (L*a*b* color system) and optical density (OD). The setting conditions for the above measurement are a viewing angle of 2 degrees and a D50 light source.

The printed film-coated tablet, the color tone of which was measured, was irradiated with cumulative visible light of 1.2 million lux by use of a xenon weather meter (Ci4000, Toyo Seiki Seisaku-sho, Ltd.). The color tone of the printed film-coated tablet irradiated with visible light was measured again using the spectrometer under the same conditions as before the visible light irradiation, and the difference in the variation (ΔE) in color tone before and after the test was compared. The comparison results are shown in Tables 1 and 2.

As a result, it was confirmed that when the printing area ratio was within the range of 30% or more and 80% or less, the variation was less than the variation when the printing area ratio was 100% (comparison target ΔE). The values shown in the "reduction rate from comparison target ΔE" column in Tables 1 and 2 indicate that the larger the absolute value, the less the variation in color tone (i.e., superior light fastness).

The "reduction rate from comparison target ΔE" in Tables 1 and 2 is a value calculated using the following formula (1):

$$(\Delta E \text{ value of each example/value of comparison target } \Delta E) - 1 \ (\%) \ldots (1)$$

The evaluation criteria in Tables 1 and 2 are shown below.

Regarding light fastness, if the amount of reduction from the comparison target ΔE was within the range of more than -20% and 0% or less, the light fastness was not significantly different from that of the solid printed image, and was therefore rated as "poor."

Further, if the amount of reduction from the comparison target ΔE was within the range of more than -40% and -20% or less, it had light fastness compared to the light fastness of the solid printed image, and was therefore rated as "fair."

Further, if the amount of reduction from the comparison target ΔE was -40% or less, the light fastness was superior to that of the solid printed image, and was therefore rated as "good."

In the present example, if the light fastness was rated as "good" or "fair," it was considered to be acceptable.

As is clear from the results in Tables 1 and 2, when the ratio of the area of a thinning printed image printed while performing thinning printing to the area of a solid printed image printed without performing thinning printing is defined as a printing area ratio (area of thinning printed image printed while performing thinning printing/area of solid printed image printed without performing thinning printing), if the printing area ratio is within the range of 30% or more and 80% or less, and the ink used for printing is an edible ink containing an edible dye, the light discoloration and light fading of the printed image can be sufficiently prevented, and the light fastness of the printed image can be improved.

The decrease rate of the area of the printed part (printing area ratio) and the decrease rate of the OD value are in an equal relationship. For example, the OD value when the area of the printed part is 80% is about 80% of the OD value when the area of the printed part is 100%, and the OD value when the area of the printed part is 60% is about 60%. On the other hand, the decrease rate of the total amount of colorants and the decrease rate of the OD value are not necessarily in an equal relationship. For example, when Comparative Example 2 is compared with Comparative Example 4, in Comparative Example 2, the total amount of colorants is 4 wt% of the entire ink, and the OD value is 0.86, whereas in Comparative Example 4, the total amount of colorants is 2.4 wt% of the entire ink, and the OD value is 0.70. Thus, in comparison with Comparative Example 2, in Comparative Example 4, the total amount of colorants is equivalent to 60%, whereas the OD value is about 80%. This is due to the fact that the increase in the OD value decreases as the total amount of colorants increases.

[Reference Signs List]

1: Tablet substrate
3: Printed image
5: Printed uncoated tablet
7: Film-coated layer
9: Printed film-coated tablet
11: Uncoated tablet printed image
13: Film-coated tablet printed image (two-dimensional barcode)
A: Solid printed part
B: Dot printed part
b: Dot
c: Broken line indicating virtually provided printed area

## Claims

1. A tablet **characterized in that**

   when a ratio of the total area of a plurality of printed parts printed while performing thinning printing to the area of a printed part printed without performing thinning printing (total area of printed parts printed while performing thinning printing/area of printed part printed without performing thinning printing) is defined as a printing area ratio, the printing area ratio is within the range of 30% or more and 80% or less,
   an ink used for printing is an edible ink, and
   the edible ink comprises an edible dye.

2. The tablet according to claim 1, wherein the edible dye comprises any one of red No. 2, red No. 3, red No. 40, red No. 102, red No. 104, red No. 105, red No. 106, yellow No. 4, yellow No. 5, green No. 3, blue No. 1, blue No. 2, and sodium copper chlorophyllin.

3. The tablet according to claim 1 or 2, wherein the tablet is a film-coated tablet.

4. The tablet according to any one of claims 1 to 3, wherein the printed image has an optical density (OD value) of 0.3 or more.

5. The tablet according to any one of claims 1 to 4, wherein the edible ink is an inkjet ink.

6. The tablet according to any one of claims 1 to 5, wherein

   the edible ink comprises the edible dye and a discoloration inhibitor, and
   the discoloration inhibitor is reduced isomaltulose, lactose, or cellobiose.

7. A printed matter **characterized in that** when a ratio of the total area of a plurality of printed parts printed while performing thinning printing to the area of a printed part printed without performing thinning printing (total area of printed parts printed while performing thinning printing/area of printed part printed without performing thinning printing) is defined as a printing area ratio, the printing area ratio is within the range of 30% or more and 80% or less,

   an ink used for printing is an edible ink, and
   the edible ink comprises an edible dye.

8. The printed matter according to claim 7, wherein the edible dye comprises any one of red No. 2, red No. 3, red No. 40, red No. 102, red No. 104, red No. 105, red No. 106, yellow No. 4, yellow No. 5, green No. 3, blue No. 1, blue No. 2, and sodium copper chlorophyllin.

9. The printed matter according to claim 7 or 8, wherein the edible ink is an inkjet ink.

10. The printed matter according to any one of claims 7 to 9, wherein

    the edible ink comprises the edible dye and a discoloration inhibitor, and
    the discoloration inhibitor is reduced isomaltulose, lactose, or cellobiose.

11. An edible ink printing method **characterized by** comprising
    performing thinning printing so that when a ratio of the total area of a plurality of printed parts printed while performing thinning printing to the area of a printed part printed without performing thinning printing (total area of printed parts printed while performing thinning printing/area of printed part printed without performing thinning printing) is defined as a printing area ratio, the printing area ratio is within the range of 30% or more and 80% or less,

    an ink used for printing being an edible ink, and
    the edible ink comprising an edible dye.

12. The edible ink printing method according to claim 11, wherein the edible dye comprises any one of red No. 2, red No. 3, red No. 40, red No. 102, red No. 104, red No. 105, red No. 106, yellow No. 4, yellow No. 5, green No. 3, blue No. 1, blue No. 2, and sodium copper chlorophyllin.

13. The edible ink printing method according to claim 11 or 12, wherein the edible ink further comprises alcohol as a solvent.

14. The edible ink printing method according to claim 13, wherein the alcohol comprises at least one of ethanol, NPA, and IPA.

15. The edible ink printing method according to claim 13 or 14, wherein the alcohol is added in an amount of 6.5 mass% or more based on the total mass of the edible ink.

16. The edible ink printing method according to any one of claims 11 to 15, wherein the edible ink further comprises a discoloration inhibitor.

17. The edible ink printing method according to claim 16, wherein the content of the discoloration inhibitor is within the range of 0.3 mass% or more and 20 mass% or less based on the total mass of the edible ink.

18. The edible ink printing method according to claim 16 or 17, wherein the discoloration inhibitor is reduced isomaltulose, citrate, malate, lactose, or cellobiose.

19. The edible ink printing method according to any one of claims 11 to 18, wherein each printed part printed with the edible ink has a circular shape in plan view, and
    the circular shape has a diameter within the range of 17 $\mu$m or more and 170 $\mu$m or less.

20. The edible ink printing method according to any one of claims 11 to 19, **characterized by** comprising printing a tablet.

21. The edible ink printing method according to any one of claims 11 to 20, wherein the edible ink is an inkjet ink.

22. The edible ink printing method according to any one of claims 11 to 21, wherein the edible

    ink comprises the edible dye and a discoloration inhibitor, and
    the discoloration inhibitor is reduced isomaltulose, lactose, or cellobiose.

# FIG.1

(a)

A

1

(b)

b
b
} B

C

1

(c)

AREA RATIO 100%

A

INK

SUBSTRATE (TABLET) — 1

⬇ LIGHT IRRADIATION

80%DECREASE

SUBSTRATE (TABLET) — 1

DECREASE RATE=100%×80%
=80%

(d)

AREA RATIO 60%

B

b

SUBSTRATE (TABLET) — 1

⬇ LIGHT IRRADIATION

80%DECREASE

SUBSTRATE (TABLET) — b
1

DECREASE RATE=60%×80%
=48%

# FIG.2

3

5

1

FIG.3

Fig.4

φ4

11 (3)

Fig.5

MEDICINE

13

7

## INTERNATIONAL SEARCH REPORT

| International application No. |
| --- |
| **PCT/JP2023/012488** |

### A. CLASSIFICATION OF SUBJECT MATTER

***A61K 9/44***(2006.01)i; ***A61K 9/30***(2006.01)i; ***A61K 47/02***(2006.01)i; ***A61K 47/10***(2017.01)i; ***A61K 47/12***(2006.01)i;
***A61K 47/20***(2006.01)i; ***A61K 47/22***(2006.01)i; ***A61K 47/24***(2006.01)i; ***A61K 47/26***(2006.01)i; ***A61K 47/36***(2006.01)i;
***A61K 47/38***(2006.01)i; ***B41J 2/01***(2006.01)i; ***B41M 5/00***(2006.01)i; ***C09D 11/30***(2014.01)i
FI:   A61K9/44; A61K9/30; A61K47/02; A61K47/10; A61K47/12; A61K47/20; A61K47/22; A61K47/24; A61K47/26;
     A61K47/36; A61K47/38; B41J2/01 401; B41J2/01 501; B41M5/00 120; C09D11/30

According to International Patent Classification (IPC) or to both national classification and IPC

### B. FIELDS SEARCHED

Minimum documentation searched (classification system followed by classification symbols)

A61K9/44; A61K9/30; A61K47/02; A61K47/10; A61K47/12; A61K47/20; A61K47/22; A61K47/24; A61K47/26;
A61K47/36; A61K47/38; B41J2/01; B41M5/00; C09D11/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 2020-2030 A (SCREEN HOLDINGS CO LTD) 09 January 2020 (2020-01-09) | 1-5, 7-9, 11-15, 19-21 |
|   | claim 1, paragraphs [0017], [0038], [0041], [0047], [0048], [0106], [0154], fig. 1-4 | |
| Y |   | 6, 10, 16-18, 22 |
| X | JP 2021-167370 A (TOPPAN PRINTING CO LTD) 21 October 2021 (2021-10-21) | 1-5, 7-9, 11-15, 20, 21 |
|   | claims 1-7, paragraphs [0015], [0026], [0027], fig. 4 | |
| Y |   | 6, 10, 16-18, 22 |
| X | JP 2022-43886 A (TOPPAN PRINTING CO LTD) 16 March 2022 (2022-03-16) | 1-5, 7-9, 11-18, 20, 21 |
|   | claims 1-8, paragraphs [0015]-[0029], fig. 4 | |
| Y | JP 2018-100336 A (SCREEN HOLDINGS CO LTD) 28 June 2018 (2018-06-28) | 6, 10, 16-18, 22 |
|   | paragraphs [0037]-[0041] | |
| Y | JP 58-134944 A (EZAKI GLICO CO) 11 August 1983 (1983-08-11) | 6, 10, 16-18, 22 |
|   | claim 1, p. 2, lower right column, lines 5-9 | |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **07 June 2023** | **20 June 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

International application No.

**PCT/JP2023/012488**

**C.     DOCUMENTS CONSIDERED TO BE RELEVANT**

| Category* | Citation of document, with indication, where appropriate, of the relevant passages | Relevant to claim No. |
|---|---|---|
| Y | JP 2022-43884 A (TOPPAN PRINTING CO LTD) 16 March 2022 (2022-03-16) paragraphs [0024], [0035], [0037] | 6, 10, 16-18, 22 |
| Y | JP 2022-43885 A (TOPPAN PRINTING CO LTD) 16 March 2022 (2022-03-16) paragraphs [0033]-[0035] | 6, 10, 16-18, 22 |

Form PCT/ISA/210 (second sheet) (January 2015)

**INTERNATIONAL SEARCH REPORT**
Information on patent family members

International application No.

**PCT/JP2023/012488**

| Patent document cited in search report | | | Publication date (day/month/year) | Patent family member(s) | Publication date (day/month/year) |
|---|---|---|---|---|---|
| JP | 2020-2030 | A | 09 January 2020 | (Family: none) | |
| JP | 2021-167370 | A | 21 October 2021 | EP 4134240 A1 claims 1-7, paragraphs [0027], [0045], [0046], fig. 4 | |
| JP | 2022-43886 | A | 16 March 2022 | (Family: none) | |
| JP | 2018-100336 | A | 28 June 2018 | CN 110088212 A paragraphs [0044]-[0048] | |
| JP | 58-134944 | A | 11 August 1983 | (Family: none) | |
| JP | 2022-43884 | A | 16 March 2022 | (Family: none) | |
| JP | 2022-43885 | A | 16 March 2022 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6389506 B

**Non-patent literature cited in the description**

- *CHEMICAL ABSTRACTS*, 915-67-3
- *CHEMICAL ABSTRACTS*, 16423-68-0
- *CHEMICAL ABSTRACTS*, 25956-17-6
- *CHEMICAL ABSTRACTS*, 2611-82-7
- *CHEMICAL ABSTRACTS*, 18472-87-2
- *CHEMICAL ABSTRACTS*, 632-69-9
- *CHEMICAL ABSTRACTS*, 3520-42-1
- *CHEMICAL ABSTRACTS*, 1934-21-0
- *CHEMICAL ABSTRACTS*, 2783-94-0
- *CHEMICAL ABSTRACTS*, 2353-45-9
- *CHEMICAL ABSTRACTS*, 3844-45-9
- *CHEMICAL ABSTRACTS*, 860-22-0